# EUROPEAN PATENT APPLICATION

(11) **EP 3 351 214 A1**
(43) Date of publication of application: **25.07.2018**
(21) Application number: 17460043.7
(22) Date of filing: 15.07.2017
(51) Int. Cl.: A61F 2/18

(54) **MIDDLE EAR PROSTHESIS OF IMPROVED EFFECTIVENESS**

(30) Priority: 19.01.2017 PL 42025617
(71) Applicant: POLITECHNIKA WARSZAWSKA, 00-661 Warszawa (PL); Park Naukowo-Technologiczny Narzadów Zmyslów Sp. z o.o., 35-045 Rzeszów (PL)
(72) Inventor: Kwacz, Monika, 05-402 Otwock (PL); Gambin, Wiktor, 01-310 Warszawa (PL); Osinski, Dymitr, 01-634 Warszawa (PL)
(74) Representative: Woznicki, Jerzy

(57) **Abstract**

The prosthesis has the conical chamber (5) created by the wall (1) in the shape similar to a truncated cone, which is closed from the side of the wider base with a rigid disk (2) with a vibration-transferring element, formed on the outside, and a flexible element (3) located between the wall (1) and the disk (2). The narrower base is terminated with the capillary tube (4) for filling the conical chamber (5) with a liquid medium, especially saline solution. The flexible element (3) constitutes a ring-shaped springy element of flexibility similar to the flexibility of the stapes annular ligament, the inner surface of the wall (1) of the chamber is formed as a convex surface, and the disk (2) surface from the side of the chamber has a formed concave hollow, where the centre of curvature of the hollow is located in the axis of the capillary tube (4), especially in the plane of its inlet opening from the side of the chamber.

## Description

The subject of the invention is a middle ear prosthesis of improved effectiveness, designed for application in ear surgery for restoring the patient's middle ear function.

Patent publication PL 217562 discloses a chamber prosthesis of the middle ear which comprises a conical chamber created by a circular wall in the shape of the side surface of a truncated cone. The wider base of the chamber is a closed membrane connected to a rigid vibration-transferring element, and the narrower base is attached to the stapes footplate. The narrower base has a capillary opening, which can be used for filling the conical chamber with a liquid medium, especially saline solution. The rigid vibration-transferring element is attached to a rigid disk mounted in the ring-shaped fold formed in the membrane. Thus constructed prosthesis makes it possible to faithfully transfer the vibrations of the tympanic membrane to the inner ear. Before placing the prosthesis in the patient's ear, the chamber is filled with saline solution. The saline solution present in the chamber eliminates attenuation of acoustic waves related to its passage through media of various sound propagation velocities. Sound waves propagating in the fluid are focused in the capillary opening of the conical chamber in accordance with the principle of acoustic lens, and then they propagate in the perilymphatic fluid present in the inner ear vestibule in the form of a plane wave. Thanks to that, the sound waves propagating in the inner ear represent the vibrations of the tympanic membrane transferred by the rigid element attached to the membrane.

The performed preclinical tests of a prototype of the chamber prosthesis have proven that there is a risk of mechanical damage of the membrane during its packing, sterilization, transport, or during the implantation procedure. A damaged membrane does not ensure effective functioning of the prosthesis and it creates unacceptable risk to the patient's safety during its prolonged use. A consequence of damaging the membrane is a leakage of fluid from the chamber and a possibility of introducing an infection into the cochlea.

Another problem is inadequate effectiveness of functioning of the chamber prosthesis in the case of co-occurrence of stapes otosclerosis together with other pathological conditions. It has been proven experimentally that in the case of stapes annular ligament otosclerosis it is possible to achieve a very good efficiency of the chamber prosthesis and to restore hearing to physiological level, i.e. complete closure of cochlear reserve. However, in the case of co-occurrence of other pathological conditions, for example tympanosclerosis, round window niche otosclerosis, cochlear otosclerosis, mixed hearing loss, the effectiveness of the chamber prosthesis may be insufficient.

The purpose of the invention is to eliminate these flaws.

The middle ear prosthesis of improved effectiveness, comprising a conical chamber created by a wall in the shape similar to a truncated cone, which is closed from the side of the wider base with a rigid disk with a vibration-transferring element formed on the outside and a flexible element located between the wall and the disk, and the narrower base is terminated with a capillary tube for filling the conical chamber with a liquid medium, especially saline solution, according to the invention is characterized in that the flexible element constitutes a ring-shaped springy element of flexibility similar to the flexibility of the stapes annular ligament, the inner surface of the conical wall of the chamber is formed as a convex surface, and the disk surface from the side of the chamber has a formed concave hollow, where the centre of curvature of the hollow is located in the axis of the capillary tube, especially in the plane of its inlet opening from the side of the chamber.

It is preferred if the capillary tube has a slantwise cut termination on the outlet, in a plane perpendicular to the tube axis.

It is preferred if the inner surface of the disk has a ring-shaped hollow for the flexible element, formed as a groove at the edge of the disk.

It is preferred if the front surface of the chamber wall from the side of the disk has a ring-shaped hollow for the flexible element, formed as a groove.

It is preferred if the wall has sockets for blocking catches formed on the outer surface, locking the disk in relation to the chamber.

Application of the flexible element in the form of a ring, replacing the membrane, eliminates the risk of chamber unsealing and significantly improves the durability of the prosthesis.

The inner surface of the chamber wall, formed as a convex surface, eliminates turbulences of the fluid and reduces the dispersion of the acoustic wave energy inside the chamber. This results in more energy transferred to the inner ear, and leads to a greater stimulation of hearing receptors than in the case of a chamber with a flat inner surface.

The concave hollow in the disk, in comparison with a flat surface, ensures a greater volume of the transferred fluid and a better orientation and focusing of the acoustic wave. This results in a greater stimulation of hearing receptors, and leads to improved effectiveness of the prosthesis.

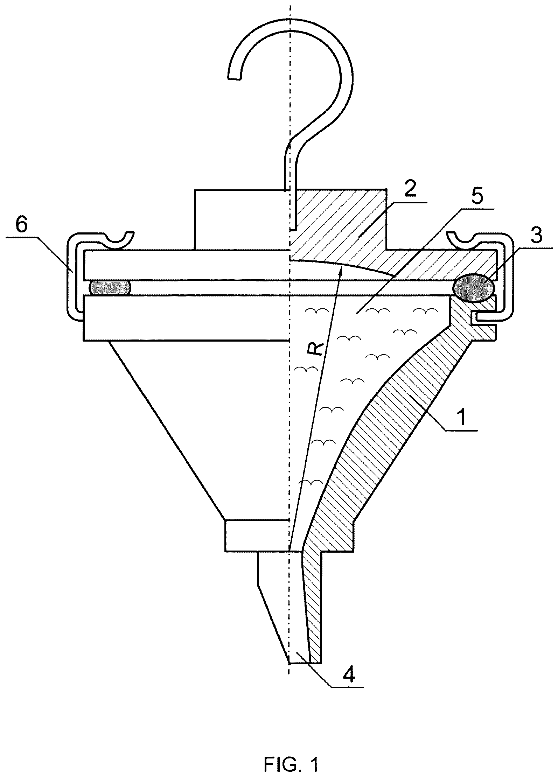

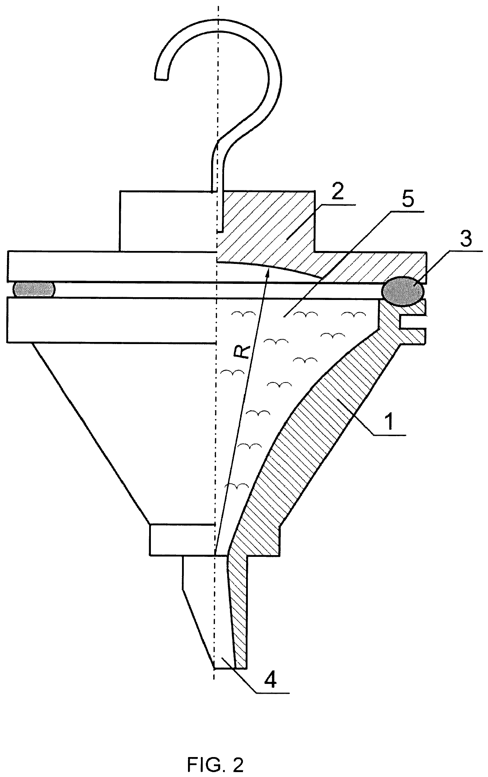

The solution according to the invention is explained in an example of its embodiment in the drawing, where fig. 1 presents the prosthesis with the disk locked by blocking catches in a half-section, and fig. 2 presents the prosthesis with the disk unlocked in a half-section.

As presented in fig. 1, 2, the prosthesis has the conical chamber 5 created by the wall 1 in the shape similar to a truncated cone, which is closed from the side of the wider base with a rigid disk 2 with a vibration-transferring element, formed on the outside, and a flexible element 3 located between the wall 1 and the disk 2. The narrower base is terminated with the capillary tube 4, which can be used for filling the conical chamber 5 with a liquid medium, especially saline solution. The flexible element 3 constitutes a ring-shaped springy element of flexibility similar to the flexibility of the stapes annular ligament, the inner surface of the conical wall 1 of the chamber is formed as a convex surface, and the disk 2 surface from the side of the chamber has a formed concave hollow. The centre of curvature of this hollow is located in the axis of the capillary tube 4, especially in the plane of its inlet opening from the side of the chamber. The capillary tube 4 has a slantwise cut termination on the outlet, in a plane perpendicular to the tube axis. The inner surface of the disk 2 has a ring-shaped hollow for the flexible element 3, formed as a groove at the edge of the disk 2. The front surface of the wall 1 from the side of the disk 2 has a ring-shaped hollow for the flexible element 3, formed as a groove. The wall 1 has sockets for the blocking catches 6 formed on the outer surface, shown in fig. 1, locking the disk 2 in relation to the chamber. The new flexible element 3 in the form of a ring, replacing the membrane, is located in ring-shaped hollows formed in the disk 2 and in the front surface of the chamber. This allows precise positioning of the flexible element 3, and protects the element from shifting. At the stage preceding the implantation procedure the disk 2 is locked in relation to the chamber using the blocking catches 6.

After the prosthesis is fastened in the patient's ear and the vibration-transferring element is connected to the patient's anvil, the blocking catches are removed. The capillary tube 4 connects the chamber with the space of the inner ear filled with perilymphatic fluid. The movement of the rigid disk 2 induces vibrations of the saline solution present in the chamber. Sound waves propagating in the fluid are focused in the inlet opening of the capillary tube 4, leading to the inner ear. A planar wave propagating in the perilymphatic fluid present in the vestibule of the inner ear will be created on the outlet side of the tube. This wave will faithfully represent the vibrations of the rigid disk 2.

The inner surface of the chamber wall, formed as a convex surface, eliminates turbulences of the fluid and reduces the dispersion of the acoustic wave energy inside the chamber. This results in more energy transferred to the inner ear, and leads to a greater stimulation of hearing receptors than in the case of a chamber with a flat inner surface. The concave hollow in the disk, in comparison with a flat surface, ensures a greater volume of the transferred fluid and a better orientation and focusing of the acoustic wave. This results in a greater stimulation of hearing receptors, and leads to improved effectiveness of the prosthesis. The slantwise cut outlet of the capillary tube orients the planar wave propagating in the vestibule of the inner ear towards hearing receptors. This results in a greater stimulation of hearing receptors.

## Claims

1. A middle ear prosthesis of improved effectiveness, comprising a conical chamber created by a wall in the shape similar to a truncated cone, which is closed from the side of the wider base with a rigid disk with a vibration-transferring element formed on the outside and a flexible element located between the wall and the disk, and the narrower base is terminated with a capillary tube for filling the conical chamber with a liquid medium, especially saline solution, **characterized in that** the flexible element (3) constitutes a ring-shaped springy element of flexibility similar to the flexibility of the stapes annular ligament, the inner surface of the conical wall (1) of the chamber (5) is formed as a convex surface, and the disk (2) surface from the side of the chamber has a formed concave hollow, where the centre of curvature of the hollow is located in the axis of the capillary tube (4), especially in the plane of its inlet opening from the side of the chamber.

2. The prosthesis according to claim 1, wherein the capillary tube (4) has a slantwise cut termination on the outlet, in a plane perpendicular to the tube axis.

3. The prosthesis according to claim 1, wherein the inner surface of the disk (2) has a ring-shaped hollow for the flexible element (3), formed as a groove at the edge of the disk (2).

4. The prosthesis according to claim 1, wherein the front surface of the wall (1) from the side of the disk (2) has a ring-shaped hollow for the flexible element (3), formed as a groove.

5. The prosthesis according to claim 1, wherein the wall (1) has sockets for blocking catches (6) formed on the outer surface, locking the disk (2) in relation to the chamber.
